## Europäisches Patentamt
## European Patent Office
## Office européen des brevets

(19)

(11) Publication number: **0 095 524**
A1

# EUROPEAN PATENT APPLICATION

(21) Application number: 82107965.4

(22) Date of filing: 30.08.82

(51) Int. Cl.³: **A 01 N 59/00, A 61 L 2/18**
// G02C13/00

(30) Priority: 01.06.82 JP 93358/82

(43) Date of publication of application: 07.12.83
Bulletin 83/49

(84) Designated Contracting States: CH DE FR LI NL SE

(71) Applicant: Hoya Lens Corporation, 25, Kowada Itsukaichi-machi Nishitama-gun, Tokyo (JP)

(72) Inventor: Hiranuma, Masahiro, 500-6, Narahashi-3-chome, Higashiyamato-shi (JP)
Inventor: Tsukamoto, Akimichi, 15-1, Honamanuma-1-chome, Suginami-ku Tokyo (JP)
Inventor: Tsuchiya, Makoto, 132-1, Otsu Sekimachi-1-chome, Nerima-ku Tokyo (JP)

(74) Representative: Patentanwälte Henkel, Pfenning, Feiler, Hänzel & Meinig, Möhlstrasse 37, D-8000 München 80 (DE)

(54) A soft contactlens preserving composition.

(57) A novel composition for preserving a soft contactlens is provided, which consists basically of sodium chloride, boric acid and borax, which is so formulated as to be in the proportion of about 0.4 – about 0.8 (preferably 0.55–0.65) w/v% of Nacl and about 0.3 – about 1.0 (preferably 0.5–0.6 w/v% in total of the latter two, when the composition is dissolved in a prescribed amount of pure water. The present composition having preferably EDTA (disodium salt) and optionally an antiseptic, a buffer agent, etc. in addition to the above 3 ingredients is more useful for providing a soft contactlens preservative solution than those conventional in causing no change in soft contacelens parameter as well as having no toxicity nor stimulation to the eyes. Especially the granular form of the present composition is convenient since it is readily soluble in water as compared with the tablet form.

0095524

A SOFT CONTACTLENS PRESERVING COMPOSITION

This invention relates to a soft contactlens preserving composition comprising boric acid, borax and sodium chloride as essential components.

Hitherto, what has been known as a soft contactlens preservative includes the physiological saline solution and solutions having further added therein such antiseptic agent and buffer substance as thimerosal, chlorohexidine, digluconate, methylparaben, propylparaben and sorbic acid, and also tablets which are used for preparing a preservative solution by dissolving a prescribed quantity of sodium chloride in a prescribed quantity of purified water or distilled water. These, however, are not given such considerations as to the physical preperties of the lacrima, to the change in the parameters of soft contactlenses under pH and osmotic pressure, and also to the property readily adsorbing chemical substances, especially organic compounds, which constitutes a weak point as well as a feature of soft contactlenses. Therefore, these conventional preserving agents have had such shortcomings that when used, they often stimulate eyes or change parameters of a soft contactlens. As for the tables, they presented a disadvantage of inconvenience to use, for it takes a fairly long time to dissolve them in a prescribed quantity of purified water or distilled water.

As the result we have devoted ourselves to the research in due consideration of the above mentioned matters, we attained this invention, discovering a safe preserving composition for soft contactlenses, which does not stimulate eyes and not give any change in the parameters of a soft contact lens.

Thus, a purpose of this invention is to provide a safe preserving composition for soft contactlenses, which contains as essential components boric acid, borax and sodium chloride, and which has the pH and osmotic pressure equal to those of the lacrima, and also does not affect the lens parameters, when dissolved in a prescribed quantity of purified water or distilled water.

The content in purified water or distilled water of boric acid and borax used in this invention after the both have been dissolved is in total from about 0.3 w/v% to about 1.0 w/v%, preferably from 0.5 w/v% to 0.6 w/v%. If the total quantity of boric acid and borax exceeds 1.0 w/v%, sometimes it stimulates eyes. If the quantity is less than 0.3 w/v%, the lens parameters apt to change. Besides, if the quantity exceeds 0.5 w/v%, a bacteriostatic action by boric acid and borax will occur to give a desirable advantage as a preservative solution for soft contactlenses. And if the

- 3 -                      0095524

above content of boric acid and borax is less than 1.0 w/v%, it does not cause any toxicity at all, when dropped in eyes.

The content in purified water or distilled water of sodium chloride used in this invention after they have been dissolved are from about 0.4 w/v% to about 0.8 w/v%, preferably from 0.55 w/v% to about 0.65 w/v%.

The content in purified water or distilled water of disodium salt of ethylenediamine tetraacetic acid (EDTA) preferably used in this invention it is from 0.05 w/v% to 0.15 w/v%, preferably from 0.08 w/v% to 0.12 w/v%. This addition of disodium salt of EDTA to the soft contactlens preserving composition of this invention gives advantages to make the preservative solution more effective by masking the metallic ions contained in the lacrima, which ions may give an injurious effect on soft contactlenses, and in addition by promoting the bacteriostatic action of boric acid.

The granular preserving agent is prepared so that when it has been dissolved in purified water or distilled water, the pH and osmotic pressure will become 7.3 and 295 mOsm respectively, which are equal to those of the lacrima. The granular agent is used by dissolving it in a prescribed quantity of purified water or distilled water.

To the preserving composition of this invention may be added an appropriate binders for formulation of the granules and also suitable sterilizers such as thymerosal and parabens.

The invention will now be described more in detail by way of example.

Example

1.3 g in the total weight of a granular preservative agent was prepared by formulating boric acid 39 wt.%, borax 7 wt.%, disodium salt of ethylenediamine tetraacetic acid 8 wt.%, and sodium chloride 46 wt.%, in the ratio thereof. The granular agent was dissolved in 100 ml of purified water, at which its pH was 7.3 and its osmotic pressure was 295 mOsm.

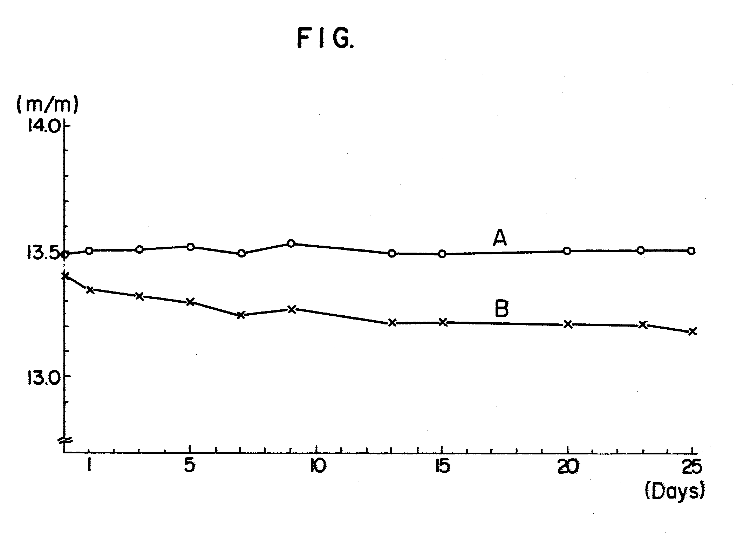

In to the preservative solution thus prepared a new soft contactlens was dipped, and change of lens parameters was measured every day when the preservative solution was renewed. At the same time, parameter change of the other one of the same type soft contactlens dipped in a physiological saline solution was measured for composition. The results are graphically illustrated in Fig. 1. In the graph, the ordinate represents diameter in mm of soft contactlens and the abscissa represents days of dipping soft contactlens. The test result in case of dipping in the preservative solution of Example 1 is indicated by line A and that in case of dipping physiological saline solution is shown by line B.

0095524

Example 2.

The preservative solution obtianed in Example 1 and a physiological saline solution as control were respectively dropped into the respective right and left eyes of a rabbit with three drops at one time every day for one month, and occurence of eye stimulus was observed. In the result no eye stimulus such as inflammation in the rabbit eyes was found in both cases of the preservative solution and the physiological saline solution.

Example 3.

Regarding the preservative solution obtained in Examle 1 the number of general living bacteria was measured. As the result, the number of living bacteria in the preservative solution was 5 - 10/ml, immediately after the dissolution of the granular preservative agent, but on the second day after the dissolution the said number decreased to 0 - 2/ml.

Example 4.

The granular preservative agent made up accordsing to the content ratio of Example 1 into the total weight of 1.3 g and a tablet prepared by the same manner into the total weight of 1.3 g were tested on their solubilities when they were dissolved in 100 ml of purified water. The result is shown in Table 1.

Table 1.

| | Time required for dissolution | |
| --- | --- | --- |
| | Not stirred | Stirred |
| Granule | 5 - 10 min | 1 - 2 min |
| Tablet | More than 10 min | 4 - 5 min |

- 7 -

What is claimed is:

1.    A soft contactlens preserving composition consisting basically of sodium chloride, boric acid and borax, which is so formulated as to be in the proportion of from about 0.4 to about 0.8 w/v% of Nacl and from about 0.3 to about 1.0 w/v% in total of boric acid and borax, when the composition is dissolved in a prescribed amount of pure water.

2.    The composition according to Claim 1, wherein said proportion is preferably from 0.55 to 0.65 w/v% of Nacl and preferably from 0.5 to 0.6 w/v% in total of boric acid and borax.

3.    The soft contactlens preserving composition according to Claims 1 or 2, which further contains disodium salt of ethylenediamine tetraacetic acid.

4.    The soft contactlens preserving composition according to Claims 1, 2 or 3, which is in the granular form.

5.    A soft contactlens preserving composition according to Claim 4, whose pH and osmotic pressure are so controlled as to be equal to those of the lacrima when said composition in the granular form is dissolved in a prescribed quanaity of purified water of distilled water.

6.    A soft contactlens preserving composition according  to Claims 4 to 5, characterized in that, when a contactlens is preserved in the preservative solution having dissolved therein said granular composition in a prescribed quantity of purified water or distilled water, the parameters of said contactlens substantially does not change.

0095524

FIG.

(Days)

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | P. LUMBROSO et al.: "Dictionnaire des produits d'entretien en contactologie", 1st edition, 9th April 1982, la source d'or, Marsat, FR.<br>* Pages 149-150 "Vitacontact" * | 1,2,5 6 | A 01 N 59/00<br>A 61 L 2/18 //<br>G 02 C 13/00 |
| | --- | | |
| Y | P. LUMBROSO et al. | 3 | |
| | --- | | |
| A | US-A-4 031 209 (J.Z. KREZANOSKI)<br>* Column 4, lines 5-52; examples I,II * | 3 | |
| | --- | | |
| A | US-A-2 547 653 (B.R. MINNIS et al.)<br>* Column 1, lines 37-39 * | 1 | |
| | ----- | | TECHNICAL FIELDS SEARCHED (Int. Cl. 3) |
| | | | A 61 L 2/18<br>A 01 N 59/00 |

The present search report has been drawn up for all claims

| Place of search | Date of completion of the search | Examiner |
|---|---|---|
| THE HAGUE | 22-04-1983 | PELTRE CHR. |